# EUROPEAN PATENT SPECIFICATION

(11) **EP 3 300 634 B1**
(45) Date of publication and mention of the grant of the patent: **13.09.2023**
(21) Application number: 17194374.9
(22) Date of filing: 02.10.2017
(51) Int. Cl.: A47C 31/00, A47C 29/00, A61N 1/16, H05K 9/00

(54) **ANTI-RADIATION ANTI-SMOG MEDICAL MATTRESS**
MEDIZINISCHE ANTI-STRAHLUNGS-ANTI-SMOG-MATRATZE
MATELAS MÉDICAL ANTI-RAYONNEMENT ANTI-BROUILLARD

(30) Priority: 30.09.2016 SK 500622016
(43) Date of publication of application: 04.04.2018
(73) Proprietor: ADITEC Slovakia s.r.o., 841 04 Bratislava (SK)
(72) Inventor: Revaj, Pavol, 029 44 Rab a (SK)
(74) Representative: Majlingova, Marta

(56) References cited:
- CN-A- 105 496 087
- CN-U- 202 941 740
- CN-U- 204 091 484
- CN-U- 204 445 015
- DE-U1- 29 909 043
- DE-U1-202004 000 722
- DE-U1-202007 003 714
- JP-A- 2004 277 847
- KR-A- 20160 045 648
- US-A- 4 681 591

## Description

### Technical field

The invention relates to a mattress having a construction suitable into a bed frame for eliminating electric charge, electrostatic and electromagnetic fields and waves that all can be considered electro-smog in relaxation zones of houses and flats.

### State of the art

Electro-smog is particularly dangerous in bedrooms. Significantly reduced melatonin hormone production (less by 40 per cent) under the effect of electro-smog has been proven. It is important to know it is necessary to build a completely closed space from electromagnetically effective materials (electrically conductive, or absorbing), a so called "Faraday's cage" for high-quality shading off of radio electromagnetic radiation (i.e. base station transmitters of mobile operators, mobile telephones, neighbouring wireless DECT telephones, Wi-Fi, etc.) or low-frequency radiation (electrical installation, high-voltage wiring, etc.).

Various sources of electromagnetic fields and electromagnetic waves, including those not exceeding the established permitted limits may result in potentially harmful biological effects and may have negative influence on human body, in particular on brain and neural systems that are controlled and operate in the body by means of electromagnetic signals of very low intensity. Said communication systems may be damaged and stressed in particular by generating electromagnetic pulse (EMP) of surrounding electric devices, wiring and appliances. In addition to said symptoms the undesirable artificially induced EMP can seriously disrupt communication and differentiation of cells as well as protein synthesis.

The undesired electromagnetic field may damage the function of human body regulatory circuits, and, as a consequence, it may lead to energy blocking similar to damaging energy management. In the long term it damages the immune system, and thus prepares foundation for acute chronic diseases. The individual EMP components \around us can be measured by a sensitive and suitable device. Various types of electromagnetic waves, which to some extent reach the rooms designated for sleeping, are constantly travelling through an atmosphere, it should be noted that also in conductive channels under the ground.

Majority of prior art documents deals with a mattress construction in terms of ventilation and firmness depending on the position of certain body parts on the mattress as well as anti-microbial treatment of the covering fabric.

SK document 4548 U describes a mattress cover for reducing the effect of electric and electrostatic fields, its subject-matter is that it consists of a fabric containing woven carbon fibres forming a dense grid, where said fibres are conductively connected to the contact component put into the fabric and a ground cable containing a protection resistor is connected to the contact component and plugged through an adaptor into an electric socket.

Examples of known anti-radiation anti-smog mattresses are disclosed in DE202004000722U1, CN204445015U, CN105496087A, DE29909043U1, KR20160045648A, JP2000277847A, US4681591A, CN204091484U, CN202941740U and DE202007003714U1.

Creating an anti-radiation anti-smog medical mattress that in addition to guaranteeing the correct body position of its user would also consist of electromagnetically effective materials (electrically conductive or absorbing materials) in order to reliably eliminate or reduce intensity and influence of electromagnetic, geopathogenic fields as well as other negative effects influencing physical and mental health and dissipate them from the user's body, was an objective of the invention. Said negative effects that are everywhere around us, although we do not see or feel them, they have impact on us and cause the following symptoms: e.g. headache, high blood pressure, sleepiness and nausea, heart pounding, eye irritation, limb trembling.

### Subject-matter of the invention

The aforementioned negative effects of electromagnetic smog on human body are removed to a large extent by an anti-radiation anti-smog medical mattress that eliminates harmful effects of electromagnetic fields and electromagnetic waves in accordance with the present invention, comprising an outer covering that envelopes the arranged inner layers of the mattress made of natural or synthetic latex or another type of natural and/or synthetic foam, such as polyurethane foams, viscoelastic foams or a mixture thereof and optionally containing various types of springs as a support core. A conventional mattress consists of a lying surface - top surface, side areas and a bottom surface.

The present invention provides an anti-radiation anti-smog medical mattress as defined in claim 1. Preferred features of the invention are set out in the dependent claims.

In accordance with another preferred embodiment the vertical portion of the conductive construction of the superstructure can be reclined to a horizontal plane of the mattress or to the side areas of the mattress when the mattress is not used.

The material for screening off electromagnetic waves and/or electromagnetic fields contains carbonised polyester wool and/or metallised polypropylene wool for protection against low-frequency electromagnetic fields.

According to another embodiment, the material for screening off electromagnetic waves and/or electromagnetic fields comprises an amorphous cobalt alloy containing cobalt, iron, molybdenum, niobium, silicon, boron and polyester lamination in the form of fibres.

According to a preferred embodiment a material layer for screening off electromagnetic waves and/or electromagnetic fields consists of metallised polyester containing compact woven screen-off netting.

According to another preferred embodiment the covering is made of a material against high-frequency and low-frequency electromagnetic fields.

The covering may comprise at minimum one material selected from the following group: cotton, polyester, spandex, nylon or polyamide in the form of fibres and/or at minimum one substance selected from the following group: copper, silver, graphite, stainless steel alloy in the form of fibres or in the form of a thin layer on the fibres cited in the aforementioned former group.

The advantage of the mattress in accordance with the present invention is that it screens off electromagnetic smog from the environment above the lying surface of the mattress and guarantees sleep of people in hygienic conditions without disturbing effects of electromagnetic waves and/or electromagnetic fields, as well as protection against tiresome insects.

### Brief description of the pictures on the drawings

The attached Figs. 1, 1a and 2 and 2a depict mattresses provided with a conductive construction in accordance with the present invention. Figs. 3 to 16a illustrate examples of cross-sections and details of mattresses.

### Exemplary embodiment

### Example 1

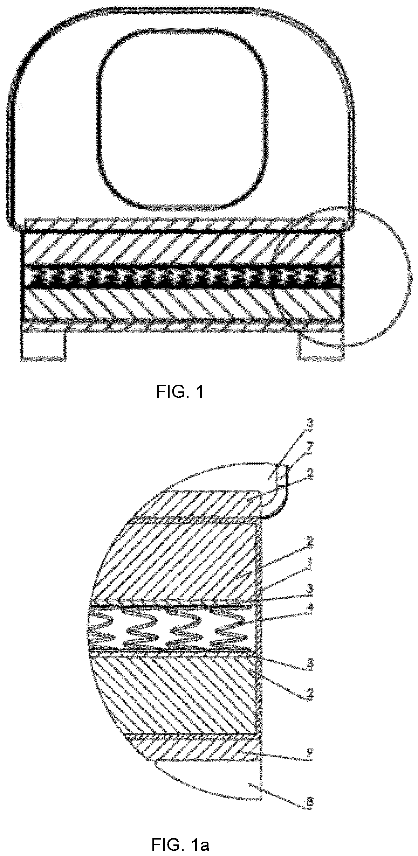

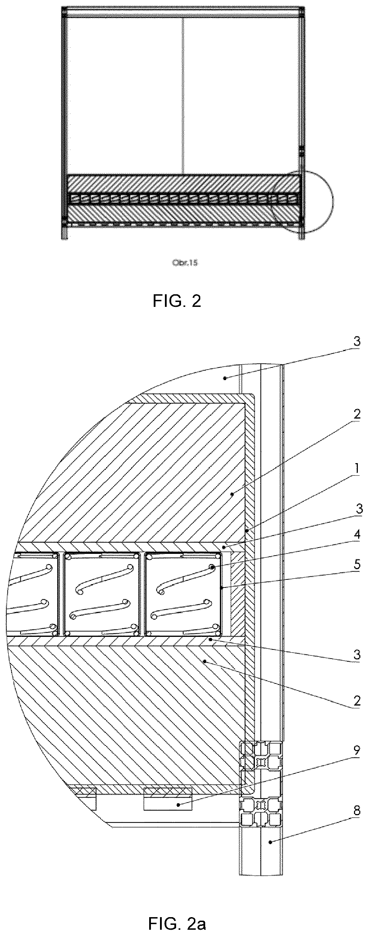

Figs 1, 1a, 2 and 2a depict mattresses s, which top horizontal surface edge is provided with conductive construction 7 of the superstructure which horizontal portion is positioned in the covering or under the covering. The vertical portion of the conductive construction 7 of the superstructure is attached to the horizontal portion of the conductive construction 7 of the superstructure in order to insert, lay, grip or attach material layer 3 for screening off electromagnetic waves and/or electromagnetic fields surrounding the top horizontal mattress surface. The vertical portion of the conductive construction 7 of the superstructure is created in the form of one arc (Fig. 1) or rectangle (Fig. 2), which can be reclined into the horizontal surface plane, i.e. lying surface of the mattress or a side surface of the mattress. The conductive construction 7 of the superstructure in the form of the arc contains a closable opening depicted on Fig. 1. Reference signs 8 and 9 represents a bed grate 9 and a bed leg 8.

### Example 2

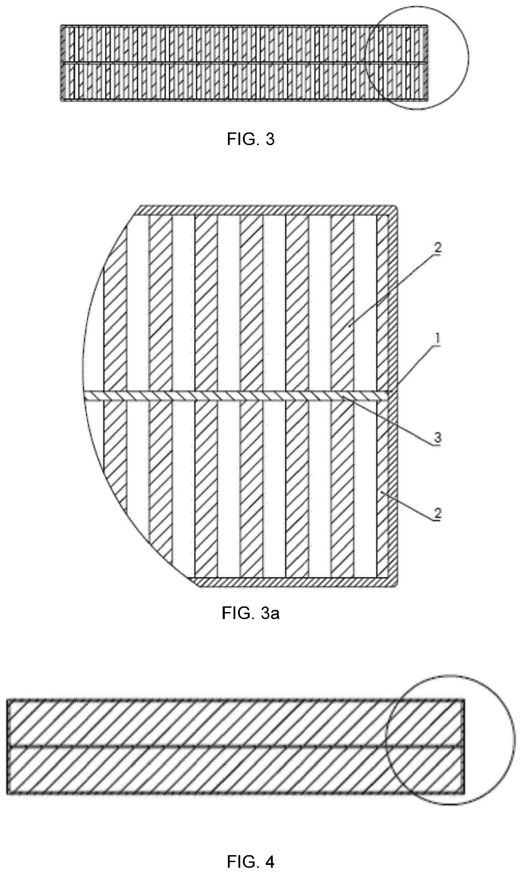

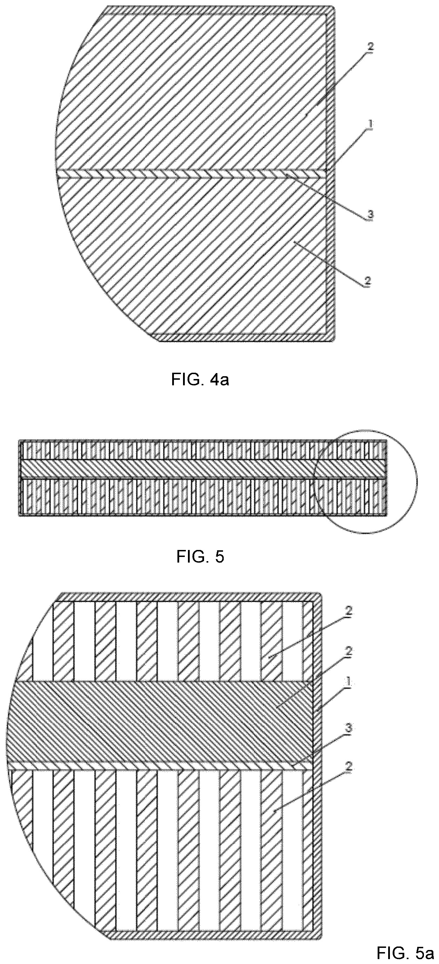

Figs 3a and 4a illustrate mattress details from Fig. 3 and Fig. 4 consisting of covering 1, of support core 2 consisting of natural latex and air channels on Fig. 3a and of support core 2 consisting of synthetic latex depicted on Fig. 4a. Layer 3 of the material serving to screen off electromagnetic waves and/or electromagnetic fields is positioned in the centre between two latex layers. Layer 3 consists of compact woven screen-off netting made of heavy metallised polyester for protection against high-frequency and low-frequency electromagnetic fields. The mattress surface is provided with covering 1 that may consist of e.g. cotton.

### Example 3

Figs. 5 and 5a illustrate a cross-sectional view of the mattress and its detail, wherein support core 2 consists of a combination of natural and synthetic latex, positioned between which is layer 3 of the material for screening off electromagnetic waves and/or electromagnetic fields consisting of a compact woven screen-off netting made of heavy metallised polyester for protection against high-frequency and low-frequency electromagnetic fields. The mattress surface is provided with covering 1 that may consist of e.g. combination of cotton and polyester fibres.

### Example 4

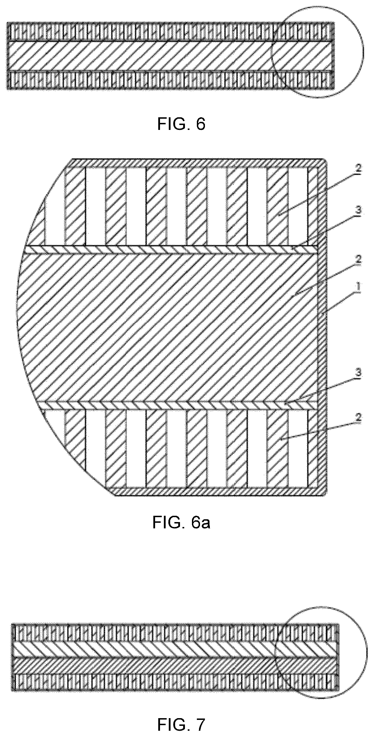

Figs. 6 and 6a illustrate a cross-sectional view of the mattress and its detail, support core 2 is formed similarly to Example 2, however, the layer of synthetic latex is provided from both sides with layer 3 of the material for screening off electromagnetic waves and/or electromagnetic fields. Layer 3 consists of compact woven screen-off netting made of heavy metallised polyester for protection against high-frequency and low-frequency electromagnetic fields. The mattress surface is provided with covering 1 that may consist of e.g. copper-, silver- or graphite- containing cotton, thereby also becoming a screening material.

### Example 5

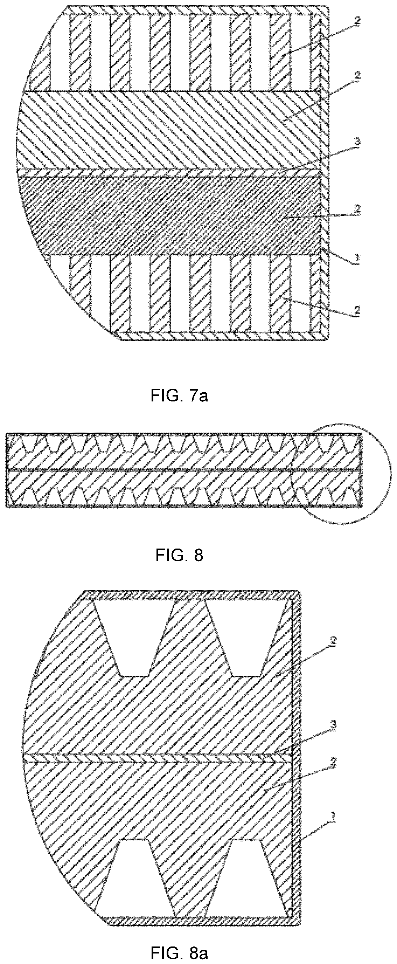

Figs. 7 and 7a illustrate a cross-sectional view of the mattress and its detail, support core 2 thereof consisting of four layers of latex and synthetic foam, between which layer 3 of the material serving to screen off electromagnetic waves and/or electromagnetic fields is inserted. Layer 3 as well as covering 1 consists of amorphous cobalt alloy containing material.

### Example 6

Figs. 8 and 8a illustrate a cross-sectional view of the mattress and its detail corresponding to construction from Fig. 1 and Fig. 1a, except that the surface of support core 2 is moulded.

### Example 7

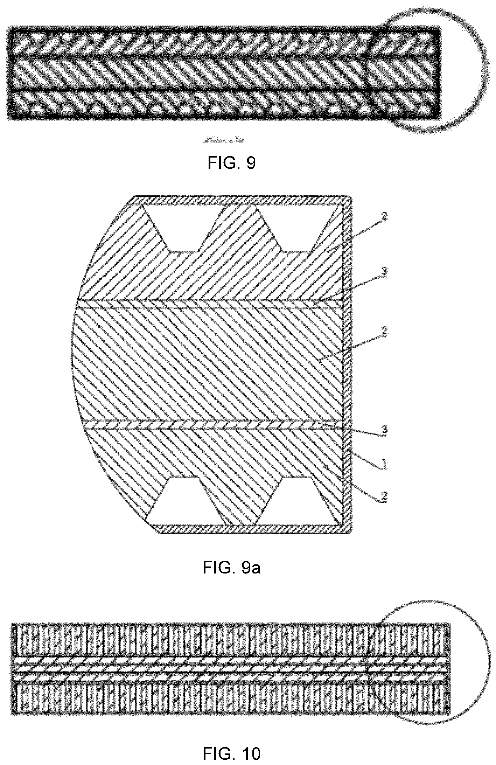

Fig. 9 and Fig. 9a depict a mattress, which support core 2 consists of 3 layers, wherein two outer layers are moulded on the surface and the central layer is from both top and bottom sides provided with layer 3 of the material for screening off electromagnetic waves and/or electromagnetic fields. Said two layers 3 may consist of one kind of material or may comprise two different kinds of screening material. Covering 1 may be made of polyamide fibre containing stainless steel fibres and may serve as a material for screening off electromagnetic waves and/or electromagnetic fields.

### Example 8

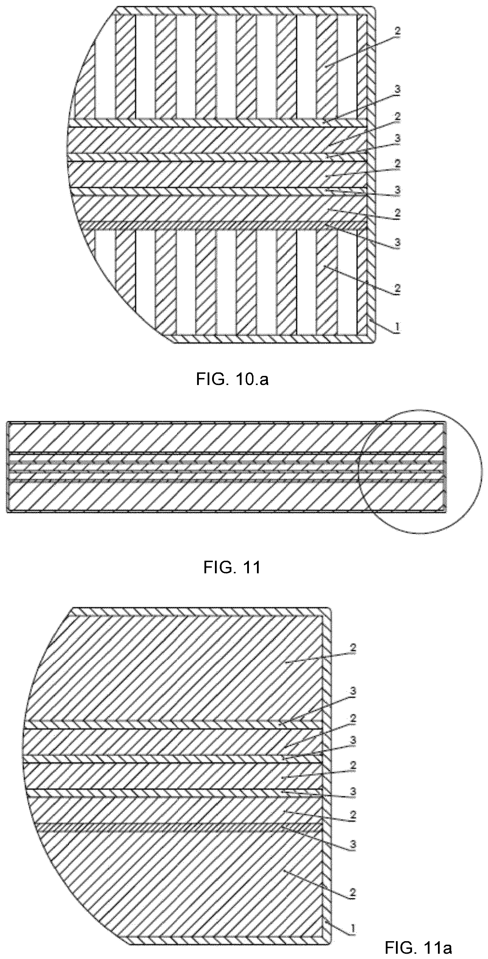

Figs. 10 and 10a, 11 and 11a illustrate mattresses that contain four layers 3 of the material serving to screen off electromagnetic waves and/or electromagnetic fields, wherein three layers 3 of the material for screening off electromagnetic waves and/or electromagnetic fields are identical and consist of compact woven screening netting made of heavy metallised polyester and one layer 3 contains amorphous cobalt alloy material with polyester lamination.

### Example 9

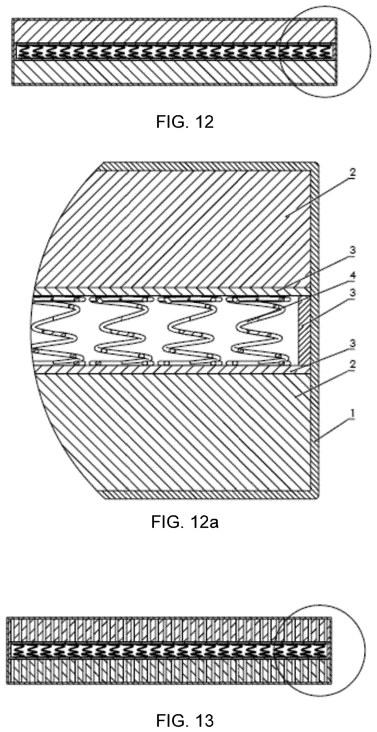

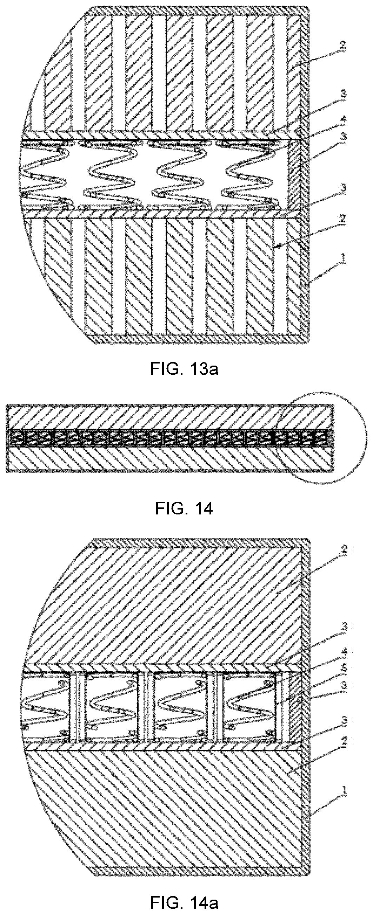

Mattress support core 2 depicted on Figs. 12 and 12a and Figs. 13 and 13a consists of central layer 4 of metal springs coated from all sides by layer 3 of the material serving to screen off electromagnetic waves and/or electromagnetic fields and two layers of latex or other natural foam, wherein said layers are mirror-arranged with respect to metal springs. Covering 1 is made of nylon and contains stainless steel fibres.

### Example 10

Mattress support core 2 depicted on Figs. 14 and 14a has identical construction as the mattress depicted on Figs. 10 and 10a, except that each spring of metal spring layer 4 is enveloped with separate casing 5 and subsequently a layer 4 of metal springs is covered with a layer 3 of the material for screening off electromagnetic waves and/or electromagnetic fields.

### Example 11

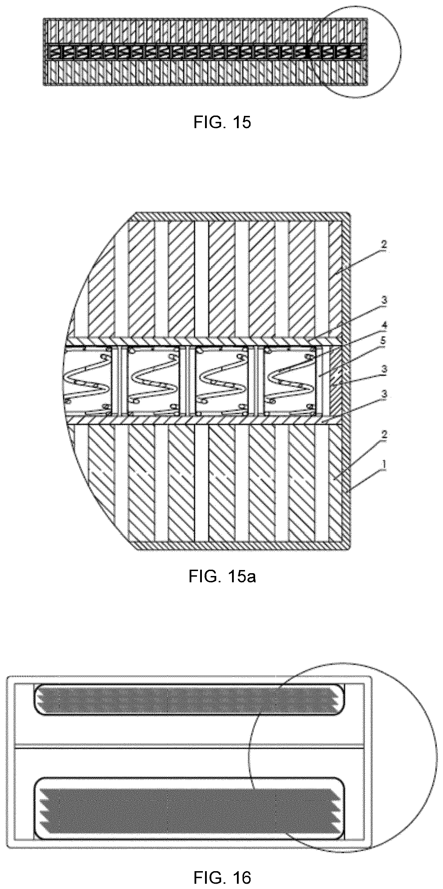

Mattress support core 2 depicted on Figs. 15 and 15a has identical construction as the mattress depicted on Figs. 11 and 11a, except that each spring of the metal spring layer 4 is coated with coating 5 and subsequently layer 4 of metal springs is wrapped with layer 3 of the material for screening off electromagnetic waves and/or electromagnetic fields.

### Example 12

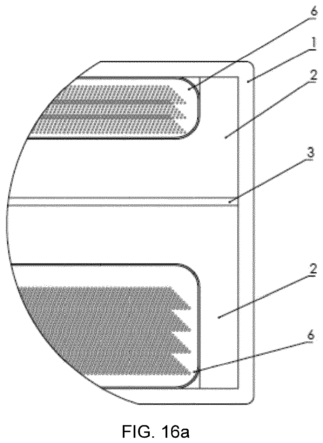

The mattress depicted on Figs 16 and 16a has a special construction. It is a double sided mattress containing a thinner and thicker removable filling, wherein there is a layer 3 of the material for screening off electromagnetic waves and/or electromagnetic fields positioned between them. Removable filling 6 containing a coating and a filler made of silicone, gel or polystyrene material, e.g. in the form of balls or in other form, is arranged in the support core 2 made of latex. The coating of the removable filling 6 may be formed by a cotton fabric or also from the cotton- and silver-containing screening material.

## Claims

1. An anti-radiation anti-smog medical mattress for elimination of harmful effects of electromagnetic fields and electromagnetic waves, said mattress containing a covering (1), that envelopes arranged layers of the mattress formed from a natural or a synthetic latex, or another type of a natural and/or a synthetic foam, optionally comprising various types of springs as a support core, the mattress comprising a lying surface, side surfaces and a bottom surface, the mattress further comprising at least one layer (3) of a material for screening off electromagnetic waves and/or electromagnetic fields, wherein said material for screening off electromagnetic waves and/or electromagnetic fields forms at least one inner layer (3), or an outer layer (3), or a combination of both layers (3) of the mattress, wherein the mattress comprising a conductive construction (7) forming a superstructure that comprises a horizontal portion and a vertical portion, wherein the horizontal portion of the conductive construction (7) of the superstructure is arranged at minimum in two edges of one horizontal mattress surface, wherein the vertical portion of the conductive construction (7) of the superstructure is attached to the horizontal portion of the conductive construction (7) of the superstructure, for insertion, laying, gripping or attaching the layer (3) of a material for screening off electromagnetic waves and/or electromagnetic fields, wherein the layer (3) of a material for screening off electromagnetic waves and/or electromagnetic fields surrounds the lying surface of the mattress and wherein the vertical portion of the conductive construction (7) of the superstructure is in the form of an arc, triangle, square or rectangle and contains a closable opening serving for entering and exiting the bed, and wherein the horizontal portion of the conductive construction (7) of the superstructure is positioned in the covering (1) or under the covering (1).

2. The anti-radiation anti-smog medical mattress according to claim 1, **characterised in that** the vertical portion of the conductive construction (7) of the superstructure is adapted for reclining into a horizontal plane of the mattress or into the side surface of the mattress.

3. The anti-radiation anti-smog medical mattress according to claim 1, **characterised in that** the layer (3) of the material for screening off electromagnetic waves and/or electromagnetic fields contains carbonised polyester wool and/or metallised polypropylene wool for protection against low-frequency electromagnetic fields.

4. The anti-radiation anti-smog medical mattress according to claim 1, **characterised in that** the layer (3) of the material for screening off electromagnetic waves and/or electromagnetic fields contains amorphous cobalt alloy in the form of fibres containing cobalt, iron, molybdenum, niobium, silicon, boron and polyester lamination.

5. The anti-radiation anti-smog medical mattress according to claim 1, **characterised in that** the layer (3) of the material for screening off electromagnetic waves and/or electromagnetic fields consists of compact woven screening netting containing metallised polyester.

6. The anti-radiation anti-smog medical mattress according to claim 1, **characterised in that** the covering (1) is made of the material against high frequency and low frequency electromagnetic fields.

7. The anti-radiation anti-smog medical mattress according to claim 1, **characterised in that** the covering (1) contains at minimum one material selected from the group of cotton, polyester, spandex, nylon or polyamide in the form of fibres and/or at minimum one substance selected from the group of copper, silver, graphite, stainless steel alloy in the form of fibres or in the form of a thin layer on the fibres listed in the former group.

## Patentansprüche

1. Medizinische Matratze gegen Strahlung und Smog zur Beseitigung der schädlichen Auswirkungen elektromagnetischer Felder und elektromagnetischer Wellen, wobei die Matratze einen Bezug (1), enthält, der die angeordneten Schichten der Matratze aus natürlichem oder synthetischem Latex oder einer anderen Art eines natürlichen und/oder eines synthetischen Schaums umhüllt, optional verschiedene Arten von Federn als Stützkern umfasst, wobei die Matratze eine Liegefläche, Seitenflächen und eine Bodenfläche umfasst, wobei die Matratze weiterhin mindestens eine Schicht (3) aus einem Material zur Abschirmung elektromagnetischer Wellen und/oder elektromagnetischer Felder umfasst, wobei das genannte Material zur Abschirmung elektromagnetischer Wellen und/oder elektromagnetischer Felder mindestens eine Innenschicht (3), oder eine Außenschicht (3), oder eine Kombination beider Schichten (3) der Matratze bildet, wobei die Matratze eine leitfähige Konstruktion (7) umfasst, die einen Aufbau bildet, der einen horizontalen Teil und einen vertikalen Teil umfasst, wobei der horizontale Teil der leitfähigen Konstruktion (7) des Aufbaus mindestens an zwei Kanten einer horizontal angeordneten Matratzenfläche angeordnet ist, wobei der vertikale Teil der leitfähigen Konstruktion (7) des Aufbaus an dem horizontalen Teil der leitfähigen Konstruktion (7) des Aufbaus befestigt ist, zum Einlegen, Legen, Ergreifen oder Befestigen der Schicht (3) des Materials zur Abschirmung elektromagnetischer Wellen und/oder elektromagnetischer Felder, wobei die Schicht (3) des Materials zur Abschirmung elektromagnetischer Wellen und/oder elektromagnetischer Felder die Liegefläche der Matratze umgibt und wobei der vertikale Teil der leitfähigen Konstruktion (7) des Aufbaus die Form eines Bogens, Dreiecks, Quadrats oder Rechtecks hat und eine verschließbare Öffnung enthält, die dem Ein- und Ausstieg aus dem Bett dient, und wobei der horizontale Teil der leitfähigen Konstruktion (7) des Aufbaus im Bezug (1) oder unter dem Bezug (1) positioniert ist.

2. Medizinische Matratze gegen Strahlung und Smog nach Anspruch 1, **dadurch gekennzeichnet, dass** der vertikale Teil der leitfähigen Konstruktion (7) des Aufbaus zum Zurücklehnen in eine horizontale Ebene der Matratze oder in die Seitenfläche der Matratze geeignet ist.

3. Medizinische Matratze gegen Strahlung und Smog nach Anspruch 1, **dadurch gekennzeichnet, dass** die Schicht (3) des Materials zur Abschirmung elektromagnetischer Wellen und/oder elektromagnetischer Felder karbonisierte Polyesterwolle und/oder metallisierte Polypropylenwolle zum Schutz vor niederfrequenten elektromagnetischen Feldern enthält.

4. Medizinische Matratze gegen Strahlung und Smog nach Anspruch 1, **dadurch gekennzeichnet, dass** die Schicht (3) des Materials zur Abschirmung elektromagnetischer Wellen und/oder elektromagnetischer Felder amorphe Kobaltlegierung in Form von Fasern enthält, die Kobalt, Eisen, Molybdän, Niob, Silizium, Bor und Polyesterlaminierung enthalten.

5. Medizinische Matratze gegen Strahlung und Smog nach Anspruch 1, **dadurch gekennzeichnet, dass** die Schicht (3) des Materials zur Abschirmung elektromagnetischer Wellen und/oder elektromagnetischer Felder aus kompakt gewebtem Abschirmnetz besteht, das metallisierten Polyester enthält.

6. Medizinische Matratze gegen Strahlung und Smog nach Anspruch 1, **dadurch gekennzeichnet, dass** der Bezug (1) aus einem Material gegen hochfrequente und niederfrequente elektromagnetische Felder besteht.

7. Medizinische Matratze gegen Strahlung und Smog nach Anspruch 1, **dadurch gekennzeichnet, dass** der Bezug (1) mindestens ein Material enthält, ausgewählt aus der Gruppe Baumwolle, Polyester, Elasthan, Nylon oder Polyamid in Form von Fasern und/oder mindestens eine Substanz ausgewählte aus der Gruppe Kupfer, Silber, Graphit, Edelstahllegierung in Form von Fasern oder in Form einer dünnen Schicht auf den in der erstgenannten Gruppe aufgeführten Fasern.

## Revendications

1. Matelas médical anti-radiation et anti-smog pour l'élimination des effets nocifs des champs électromagnétiques et des ondes électromagnétiques, ledit matelas contenant une couverture (1), qui entoure les couches disposées du matelas, formées d'un latex naturel ou synthétique ou d'un autre type de mousse naturelle et/ou synthétique, comprenant éventuellement des types divers de ressorts comme noyau de support, ledit matelas comprenant une surface de couchage, des surfaces latérales et une surface inférieure, ledit matelas comprenant également au moins une couche (3) d'un matériau destiné à retenir les ondes électromagnétiques et/ou les champs électromagnétiques, où ledit matériau destiné à retenir les ondes électromagnétiques et/ou les champs électromagnétiques forme au moins une couche (3) interne ou une couche (3) externe, ou une combinaison des deux couches (3) du matelas, où le matelas comprend une construction (7) conductrice formant une superstructure qui comporte une partie horizontale et une partie verticale, où la partie horizontale de la construction (7) conductrice de la superstructure est disposée au minimum sur deux bords d'une surface horizontale du matelas, où la partie verticale de la construction (7) conductrice de la superstructure est attachée à la partie horizontale de la construction (7) conductrice de la superstructure, afin de pouvoir insérer, poser, saisir ou fixer la couche (3) du matériau destiné à retenir les ondes électromagnétiques et/ou les champs électromagnétiques, où la couche (3) du matériau destiné à retenir les ondes électromagnétiques et/ou les champs électromagnétiques entoure la surface de couchage du matelas, et où la partie verticale de la construction (7) conductrice de la superstructure a la forme d'un arc, triangle, carré ou rectangle et contient une ouverture refermable permettant d'entrer et de sortir du lit, et où la partie horizontale de la construction (7) conductrice de la superstructure est placée dans la couverture (1) ou sous la couverture (1).

2. Matelas médical anti-radiation et anti-smog selon la revendication 1 **caractérisé en ce que** la partie verticale de la construction (7) conductrice de la superstructure est adaptée pour s'incliner dans le plan horizontal du matelas ou dans la surface latérale du matelas.

3. Matelas médical anti-radiation et anti-smog selon la revendication 1 **caractérisé en ce que** la couche (3) du matériau destiné à retenir les ondes électromagnétiques et/ou les champs électromagnétiques contient de la laine de polyester carbonisée et/ou de la laine de polypropylène métallisée pour la protection contre les champs électromagnétiques de basse fréquence.

4. Matelas médical anti-radiation et anti-smog selon la revendication 1 **caractérisé en ce que** la couche (3) du matériau destiné à retenir les ondes électromagnétiques et/ou les champs électromagnétiques contient alliage de cobalt amorphe sous forme de fibres contenant du cobalt, fer, molybdène, niobium, silicium, bore et une stratification de polyester.

5. Matelas médical anti-radiation et anti-smog selon la revendication 1 **caractérisé en ce que** la couche (3) du matériau destiné à retenir les ondes électromagnétiques et/ou les champs électromagnétiques est composée par un filet de criblage tissé compact contenant du polyester métallisé.

6. Matelas médical anti-radiation et anti-smog selon la revendication 1 **caractérisé en ce que** la couverture (1) est composée d'un matériau résistant aux champs électromagnétiques de haute et de basse fréquence.

7. Matelas médical anti-radiation et anti-smog selon la revendication 1 **caractérisé en ce que** la couverture (1) contient au moins un matériau sélectionné parmi coton, polyester, élasthanne, nylon, polyamide sous forme de fibres et/ou au moins une substance sélectionnée parmi des alliages de cuivre, argent, graphite, acier inoxydable sous forme de fibres ou sous forme d'une couche fine sur les fibres mentionnées ci-haut.
